# Europäisches Patentamt

(19) **European Patent Office**

**Office européen des brevets**

(11) Publication number: **0 133 029**
**B1**

(12) # EUROPEAN PATENT SPECIFICATION

(45) Date of publication of patent specification: **27.12.90**

(51) Int. Cl.⁵: **A 61 K 35/12**

(21) Application number: **84305099.8**

(22) Date of filing: **26.07.84**

(54) **A process for producing inhibiting agents against proliferation of malignant tumour cells, and agents produced thereby.**

(30) Priority: **28.07.83 JP 136794/83**

(43) Date of publication of application:
**13.02.85 Bulletin 85/07**

(45) Publication of the grant of the patent:
**27.12.90 Bulletin 90/52**

(84) Designated Contracting States:
**CH DE FR GB LI NL**

(56) References cited:
**EP-A-0 087 087**
**EP-A-0 102 801**
**EP-A-0 118 286**
**EP-A-0 125 125**
**EP-A-0 133 358**
**EP-A-0 136 053**

**BIOLOGICAL ABSTRACTS, vol. 66, no. 7, 1978, no. 41061, Philadelphia, Pa., US; D.L. DEWEY: "The identification of a cell culture inhibitor in a tumour extract"**

(73) Proprietor: **KOKEN LTD.**
**No. 7, Yonban-cho**
**Chiyoda-ku Tokyo (JP)**

(73) Proprietor: **Tajima, Tomoyuki**
**5-15, Yawata 6-chome**
**Ichikawa-shi Chiba-ken (JP)**

(73) Proprietor: **Nagasu, Youichiro**
**No. 9-4, Chuo 3-chome**
**Yamato-shi Kanagawa-ken (JP)**

(72) Inventor: **Tajima, Tomoyuki**
**No. 5-15, Yawata 6-chome**
**Ichikawa-shi Chiba-ken (JP)**

(74) Representative: **Deans, Michael John Percy et al**
**Lloyd Wise, Tregear & CO. Norman House**
**105-109 Strand**
**London WC2R OAE (GB)**

(56) References cited:

**BIOLOGICAL ABSTRACTS, vol. 65, 1978, no. 3219, Philadelphia, Pa., US; D.M. BURNS et al.: "Natural occurrence of an inhibitor of mammalian cell growth in human and mouse cells of normal and tumor origin"**

Courier Press, Leamington Spa, England.

# EP 0 133 029 B1

(56) References cited:

BIOLOGICAL ABSTRACTS, vol. 65, 1978, no. 34666, Philadelphia, Pa., US; G.L. WRIGHT, Jr. et al.: "Isolation of a soluble tumor-associated antigen from human renal cell carcinoma by gradient acrylamide gel electrophoresis"

BIOLOGICAL ABSTRACTS, vol. 65, no. 3, 1978, no. 16073, Philadelphia, Pa., US; J.L. MCCOY et al.: "Leukocyte migration inhibition in patients with Ewing's sarcoma by 3-M potassium chloride extracts of fresh and tissue-cultured Ewing's sarcomas"

**Description**

The present invention relates to the production of preparations for inhibiting proliferation of malignant tumour cells.

We have previously proposed in our own European Patent Application 83304845.7 (now published as EP—A—0102801) the production of an inhibiting agent against the proliferation of malignant tumour cells comprising the steps of incubating malignant tumour cells and, after removing the malignant tumour cells, extracting an inhibiting agent from the culture medium. We have found that inhibiting agents so produced appear not to show lethal effects against normal cells, but instead to exhibit an inhibiting activity against the proliferation of malignant tumour cells and to have a lethal effect specifically to malignant tumour cells.

The present invention arises from our work seeking to obtain preparations with a significantly enhanced inhibiting effect even when provided in substantially smaller doses.

In accordance with a first aspect of the present invention, we provide a process for producing a preparation for inhibiting the proliferation of malignant tumour cells, characterised in that it comprises the steps of: incubating malignant tumour cells in a culture medium; further incubating the said malignant tumour cells after adding serum protein or albumin to the culture medium; isolating the culture medium by removing said malignant tumour cells; subjecting the culture medium to ultrafiltration through a filter for the molecular weight of 10,000; and separating the fraction having a molecular weight of 10,000 or more.

In the preferred procedure described in more detail below, the malignant tumour cells are first allowed to proliferate in a growth medium until the incubator is saturated. The cells are transferred to an extracting medium and further incubated after adding serum protein or albumin. The culture medium is isolated by removing the malignant tumour cells. Ultrafiltration is conducted through an Amicon-filter to isolate the fraction having a molecular weight of 10,000 or more.

We have found that inhibiting agents against the proliferation of malignant tumour cells link with the serum protein or albumin in this procedure. The links in effect result in polymerization. We find that by this procedure we can obtain an inhibiting agent whose concentration is ten times or more as high as that of the inhibiting agent produced in our first proposal referred to hereinabove.

Since we are now able to provide inhibiting agents concentrated by ten or more times as compared with our prior proposal, a substantially enhanced inhibition can be produced against malignant tumour cells at a significantly smaller dose.

In a second and alternative aspect of the present invention, we provide an inhibiting preparation for inhibiting the proliferation of malignant tumour cells, characterised in that it comprises an agent having a molecular weight of 10,000 or more linked to serum protein or albumin and is free of components having a molecular weight of less than 10,000.

The invention is hereinafter more particularly described by way of detailed illustrative examples.

Example 1

For this experiment we employed an established cell line (hereinafter referred to as "HRC") originating from a human renal carcinoma.

For the growth medium we employed Basal Medium Eagle (hereinafter referred to as "BME") supplemented with 10% new born calf serum. The HRC cells were added and allowed to proliferate in an incubator to the extent that they were saturated. The serum was removed by washing once and the cells transferred to an extracting medium where they were further incubated in BME supplemented with 10% new born calf serum at a temperature of between 30°C and 37°C for one week.

To isolate the culture medium from the cells grown therein, the incubated culture medium was centrifuged at 3,000 rpm for 10 minutes and then filtered through a 0.45 μm Millipore filter.

The isolated culture medium was then separated into two fractions, one having a molecular weight of 10,000 or more (hereinafter referred to as "fr$\geq 10^4$"), and the other having a molecular weight less than 10,000 (referred to hereinafter as "fr$< 10^4$"), using a filter for the molecular weight of $10^4$ made by Amicon.

We shall now describe the experiments which we have performed to confirm the inhibiting effects exhibited by the product resulting from Example 1.

To a culture medium of BME supplemented with 10% new born calf serum placed in a plastic dish, some $10^4$ cells of HRC were inoculated. After incubation for 24 hours, a culture medium containing the product resulting from Example 1 was exchanged for the first culture medium, and replacement of the culture medium was carried out in the same culture medium every other day. The proliferation of the cells was determined on the sixth day.

Comparative tests were performed with fraction fr$< 10^4$ and also with the tumour cell anti-proliferation agent resulting from our prior proposal referred to hereinabove and hereinafter referred to as "untreated".

In the culture medium containing fr$\geq 10^4$, 10% or 20% new born calf serum was supplemented in order to reproduce the former concentration because the substances in fr$\geq 10^4$ were considerably concentrated. On the other hand, in the culture medium containing fr$< 10^4$, used as a control, amino acids, vitamins and glucose, being the same ingredients and used in the same concentration as contained in BME were added to complement the nutrients consumed during extraction, and also 10% or 20% new born calf serum was added. In order to provide a comparison of effect between the agent in accordance with the present invention, the control, and the untreated agent, the survival numbers of malignant tumour cells were

EP 0 133 029 B1

counted on the sixth day and the survival rate calculated as a percentage based on the cell numbers at the beginning of the determination. A measure of specific activity was produced by comparing with the untreated case after calculating the death rate by subtracting the survival rate from 100 and by calculating the death rate per unit dose by dividing the death rate by the dose employed. The experimental results set out in table 1 below were produced.

TABLE 1

| Lot | | Survival rate (%) | Concentrated degree (%) | Dose | Specific activity |
|---|---|---|---|---|---|
| | untreated | 0.2 | 1 | 6.0 | 1 |
| Lot 81 | fr≥$10^4$ | 6.6 | 17.1 | 0.35 | 16.2 |
| | fr<$10^4$ | 57 | 1 | 6.0 | 0.43 |

It will be seen from Table 1 that the inhibiting agent produced by the present invention is concentrated by at least ten times when compared with the untreated agent previously proposed. Similar inhibiting effects against the proliferation of malignant tumour cells is produced at a very much smaller dose, namely less than 1/10th of that previously required.

Example 2

HRC cells were added to Basal Medium Eagle (BME) supplemented with 10% new born calf serum and the malignant tumour cells were allowed to proliferate to saturate the incubator. An appropriate amount of bovine serum or bovine albumin was then added and allowed to stand at a temperature of 4°C for 24 hours.

The tumour cell anti-proliferation agent in accordance with the present invention was isolated by a procedure substantially the same as that described above with regard to Example 1. Similar tests were performed as before. The experimental results are shown in Table 2.

TABLE 2

| Lot | | Survival rate | Concentrated degree | Dose | Specific activity |
|---|---|---|---|---|---|
| | Untreated | 1.2 | 1 | 6 | 1 |
| Lot 101 A | fr≥$10^4$ | 7.2 | 18.2 | 0.33 | 17.1 |
| | fr<$10^4$ | 6.7 | 1 | 6 | 1 |
| Lot 101 B | fr≥$10^4$ | 10.1 | 20.0 | 0.30 | 17.8 |
| | fr<$10^4$ | 56 | 1 | 6 | 1 |

**Claims**

1. A process for producing a preparation for inhibiting the proliferation of malignant tumour cells, characterized in that it comprises the steps of: incubating malignant tumour cells in a culture medium; further incubating the said malignant tumour cells after adding serum protein or albumin to the culture medium; isolating the culture medium by removing said malignant tumour cells; subjecting the culture medium to ultra-filtration through a filter for the molecular weight of 10,000; and separating the fraction having a molecular weight of 10,000 or more.

2. A process according to Claim 1, further characterised in that the malignant tumour cells are an established cell line originating from human renal carcinoma cells.

3. A process according to Claim 1 or Claim 2, further characterized in that Basal Medium Eagle, to which 10% new born calf serum is added, is used as the culture medium.

4. A process according to any preceding claim, further characterized in that the culture medium in which the malignant tumour cells are first incubated is a growth medium therefor, and in that the culture medium in which the cells are further incubated after adding serum protein or albumin is an extraction medium to which the cells are transferred from said growth medium.

5. An inhibiting preparation for inhibiting the proliferation of malignant tumour cells, characterised in that it comprises an agent having a molecular weight of 10,000 or more linked to serum protein or albumin and is free of components having a molecular weight of less than 10,000.

4

## Patentansprüche

1. Verfahren zur Herstellung einer Zubereitung zur Hemmung der Vermehrung maligner Tumorzellen, dadurch gekennzeichnet, daß es die Stufen Inkubieren der malignen Tumorzellen in einem Kulturmedium, weiteres Inkubieren dieser malignen Tumorzellen nach Zugabe von Serumprotein oder -albumin zum Kulturmedium, Isolieren des Kulturmediums durch Entfernung dieser malignen Tumorzellen, Ultrafiltration des Kulturmediums durch ein Filter für ein Molekulargewicht von 10.000 und Abtrennen der Fraktion mit einem Molekulargewicht von größer oder gleich 10.000 umfaßt.

2. Verfahren nach Anspruch 1, dadurch gekennzeichnet, daß die malignen Tumorzellen eine etablierte Zellkultur sind, die ihren Ursprung in menschlichen Nierenkrebszellen hat.

3. Verfahren nach Anspruch 1 oder 2, dadurch gekenneichnet, daß Basal Medium Eagle, dem 10% Serum eines neugeborenen Kalbes zugegeben wurden, als Kulturmedium verwendet wird.

4. Verfahren nach einem der vorhergehenden Ansprüche, dadurch gekennzeichnet, daß das Kulturmedium, in welchem die Tumorzellen zuerst inkubiert werden, ein Wachstumsmedium für diese ist und daß das Kulturmedium, in welchem die Zellen nach der Zugabe von Serumprotein oder-albumin weiter inkubiert werden, ein Extraktionsmedium ist, in das die Zellen aus dem Wachstumsmedium überführt worden sind.

5. Hemmende Zubereitung zur Hemmung der Vermehrung maligner Tumorzellen, dadurch gekennzeichnet, daß sie ein Mittel mit einem Molekulargewicht von größer oder gleich 10.000 umfaßt, das an Serumprotein oder -albumin gebunden und frei von Bestandteilen mit einem Molekulargewicht von kleiner als 10.000 ist.

## Revendications

1. Procédé de fabrication d'une préparation inhibant la prolifération de cellules tumorales malignes, caractérisé en ce qu'il comprend les étapes suivantes: incubation de cellules tumorales malignes dans un milieu de culture approprié; incubation subséquente des mêmes cellules tumorales malignes après addition au milieu de culture de sérum protéique ou d'albumine; séparation desdites cellules tumorales malignes de leur milieu de culture; soumission du milieu résiduel à une ultra filtration au moyen d'un filtre susceptible de retenir des particles de poids moléculaire 10.000; séparation du milieu de fractions ayant un poids moléculaire égal ou supérieur à 10.000.

2. Procédé suivant la revendication 1 caractérisé en ce que les cellules tumorales malignes proviennent de cellules rénales humaines atteintes de carcinome.

3. Procédé suivant les revendications 1 et 2 caractérisé en ce que le Milieu Basal Eagle enrichi dans la mesure de 10% par du sérum de veau nouveau-né est utilisé comme milieu de culture.

4. Procédé suivant l'une quelconque des revendications 1 à 3 caractérisé en ce que le milieu de culture dans lequel les cellules tumorales malignes sont d'abord incubées, est un milieu de croissance, et en ce que le milieu de culture additionné de sérum protéique ou d'albumine dans lequel les cellules sont transférées du milieu de croissance pour y être de nouveau incubées, est un milieu extracteur.

5. Préparation pour inhiber la prolifération de cellules tumorales malignes, caractérisée en ce qu'elle contient un agent de poids moléculaire égal ou supérieur à 10.000 lié au sérum protéique ou à l'albumine et dépourvu de composants ayant un poids moléculaire inférieur à 10.000.